# EUROPEAN PATENT SPECIFICATION

(11) **EP 4 393 523 B1**
(45) Date of publication and mention of the grant of the patent: **05.08.2026**
(21) Application number: 23854198.1
(22) Date of filing: 25.07.2023
(51) Int. Cl.: A61L 27/36, A61L 27/20, A61L 27/24, A61L 27/50, A61L 27/58

(54) **CELLULAR MATRIX NERVE GRAFT FOR REPAIRING PERIPHERAL NERVE INJURY AND PREPARATION METHOD THEREFOR**
ZELLMATRIXNERVENTRANSPLANTAT ZUR REPARATUR VON PERIPHEREN NERVENLÄSIONEN UND HERSTELLUNGSVERFAHREN DAFÜR
GREFFON NERVEUX À MATRICE CELLULAIRE POUR RÉPARER UNE LÉSION DU NERF PÉRIPHÉRIQUE ET SON PROCÉDÉ DE PRÉPARATION

(30) Priority: 16.08.2022 CN 202210982152
(43) Date of publication of application: 03.07.2024
(73) Proprietor: Nantong University, Nantong, Jiangsu 226019 (CN)
(72) Inventor: LI, Meiyuan, Nantong, Jiangsu 226019 (CN); GU, Xiaosong, Nantong, Jiangsu 226019 (CN); XU, Lingchi, Nantong, Jiangsu 226019 (CN); GONG, Leilei, Nantong, Jiangsu 226019 (CN); SUN, Hualin, Nantong, Jiangsu 226019 (CN); WANG, Shanshan, Nantong, Jiangsu 226019 (CN); WANG, Hongkui, Nantong, Jiangsu 226019 (CN); QIAN, Tianmei, Nantong, Jiangsu 226019 (CN); XU, Lai, Nantong, Jiangsu 226019 (CN)
(74) Representative: Proi World Intellectual Property GmbH
(86) International application number: PCT/CN2023/109010
(87) International publication number: WO 2024/037293

(56) References cited:
- CN-A- 102 218 160
- CN-A- 103 041 450
- CN-A- 103 041 450
- CN-A- 108 310 467
- CN-A- 110 584 829
- CN-A- 112 546 303
- CN-A- 115 337 458
- JP-A- 2009 045 221
- US-A1- 2003 171 824
- US-A1- 2008 213 389
- XIURUI ZHANG ET AL: "Mesenchymal stem cell-derived extracellular matrix (mECM): a bioactive and versatile scaffold for musculoskeletal tissue engineering", BIOMEDICAL MATERIALS, INSTITUTE OF PHYSICS PUBLISHING, BRISTOL, GB, vol. 16, no. 1, 23 December 2020 (2020-12-23), pages 12002, XP020362364, ISSN: 1748-605X, [retrieved on 20201223], DOI: 10.1088/1748-605X/ABB6B3
- WANG SHENGRAN ET AL: "BMSC-derived extracellular matrix better optimizes the microenvironment to support nerve regeneration", BIOMATERIALS, ELSEVIER, AMSTERDAM, NL, vol. 280, 13 November 2021 (2021-11-13), XP086909067, ISSN: 0142-9612, [retrieved on 20211113], DOI: 10.1016/J.BIOMATERIALS.2021.121251

## Description

### BACKGROUND OF THE INVENTION

### 1. Technical Field

The invention discloses a cell matrix nerve graft for repairing peripheral nerve injury and a preparation method thereof and belongs to the technical field of medical biomaterials that can be implanted into human bodies.

### 2. Description of Related Art

The technical solutions of medical biomaterials that can be implanted into human bodies are known from CN 103041450 A, CN 108310467 A and Xiurui Zhang et al: "Mesenchymal stem cell-derived extracellular matrix (mECM): a bioactive and versatile scaffold for musculoskeletal tissue engineering". Peripheral nerve injury, which is extremely common in clinical practice, not only seriously affects the patient's quality of life, but also increases the patient's economic burden. In the prior art, microsurgical alignment suturing is usually used to repair short-distance peripheral nerve defects. Long-distance large peripheral nerve defects are repaired often by way of autologous nerve transplantation and scaffold material transplantation. Among them, autologous nerve transplantation is the gold standard for nerve transplantation and repair, which involves surgical removal of a section of autologous nerve, such as the medial or lateral forearm cutaneous nerve, ulnar nerve, femoral nerve or radial nerve branches, and then transplanting to repair the damaged nerve. Schwann cells in grafts are activated and secrete a large amount of neurotrophic factors, allowing axons to regenerate faster. However, problems such as limited donor sources, inconsistent nerve diameter matching, and permanent denervation of the donor site limit the clinical application of autologous nerve transplantation. The application of biological scaffold materials is to make nerves grow in a certain direction and prevent the occurrence of neuromas. However, the repair and regeneration process takes a long time, the nerve regeneration rate is slow, and effectors and receptors atrophy quickly.

A tissue-engineered nerve graft, which is an organic unity comprising a scaffold material, supporting cells, extracellular matrix, and nerve growth factors, can be used as a bridge to guide the damaged nerve fibers to extend from the proximal end to the distal end of the injury. However, its clinical translation faces many problems, such as the type and quantity of cells or factors, cell viability or factor activity, cell phenotype stability, treatment time, regulatory intervention and high cost.

As a viable tissue engineering graft, acellular matrix (ACM) is gradually favored by researchers and used to repair tissue injury. Decellularizing allogeneic or xenogeneic neural (or non-neural) tissue through various physical, chemical and enzymatic methods can produce a tissue-derived acellular matrix. The tissue-derived acellular matrix is a non-cellular biomaterial suitable for the preparation of nerve scaffolds and can be used as a substitute for autologous nerve grafts to repair peripheral nerve defects. Compared with scaffolds prepared with individual extracellular matrix components, tissue-derived acellular matrix scaffolds (also known as acellular nerve grafts) have a better ability to preserve the basic structure of the native tissue and promote peripheral nerve regeneration. Although tissue- or organ-derived acellular matrix scaffolds possess biochemical and physical factors that support tissue or cell growth, obtaining donor tissue of consistent quality is still a critical limitation. **In** addition, they also have some defects, such as potential immunogenicity, transfer of pathogens, uncontrolled degradation and high manufacturing costs. Therefore, the repair and regeneration after clinical peripheral nerve injury are still a huge challenge.

### BRIEF SUMMARY OF THE INVENTION

The invention is set out in the appended set of claims. The objective of the invention is to overcome the defects in the prior art and provide a cell matrix nerve graft for repairing peripheral nerve injury and a preparation method thereof, which facilitates cell adhesion and migration and promotes nerve regeneration and functional recovery.

In order to achieve the above objective, the technical solutions of the invention are as follows:

In a first aspect, the invention provides a cell matrix nerve graft for repairing peripheral nerve injury, comprising an acellular matrix and a scaffold, wherein the acellular matrix is obtained by decellularization after secretion and formation of stem cells. In this way, various important components and frameworks of the extracellular matrix are retained, which is beneficial to the adhesion of nerve cells and the guidance of axon regeneration, and accelerates the regeneration and functional recovery of peripheral nerves. The acellular matrix is wrapped around the scaffold.

In view of the first aspect, further, the stem cells are human bone marrow mesenchymal stem cells (hBMSCs), which facilitates the clinical transformation of tissue-engineered nerve grafts.

Further, the scaffold is a biodegradable scaffold, comprising chitosan and recombinant human collagen. The scaffold is characterized by good biocompatibility and degradability, and can guide the growth direction for nerve axon regeneration.

In a second aspect, the invention further provides a preparation method of the cell matrix nerve graft for repairing peripheral nerve injury according to any one of the above, including the following steps:
preparing the acellular matrix;
wrapping the acellular matrix around a scaffold to construct an initial form of the cell matrix nerve graft;
setting the initial form of the cell matrix nerve graft still at 2-6 °C for at least 24 h to allow self-assembly to form a tubular structure; and
lyophilizing the tubular structure to construct the cell matrix nerve graft. The cell matrix nerve graft prepared by this method does not contain exogenous toxic substances brought in by the preparation process, has good biocompatibility, biodegradability, and good mechanical properties.

**In** view of the second aspect, further, the acellular matrix is rolled into multiple layers with the scaffold as the axis, wrapped around the biodegradable scaffold, and can self-assemble to form a tubular structure, which provides necessary pathways for the growth of nerve cells and takes the effect of guidance and oriented growth.

Further, the acellular matrix is rolled up to 6, 9 or 12 layers with the biodegradable scaffold as the axis, and wrapped around the biodegradable scaffold.

Further, the tubular structure is lyophilized at -80 °C.

Further, the acellular matrix is prepared as follows:
diluting hBMSCs, seeding the hBMSCs into a large dish, and when the cell saturation reaches 90% or above, adding a serum-free culture medium supplemented with ascorbic acid to stimulate the stem cells for 7-14 days to promote the cells to secrete extracellular matrix; and
flushing with a phosphate buffer, adding sterile ultrapure water for hypotonic lysis, removing the sterile ultrapure water, adding a cell lysis buffer for extraction, and then flushing with the phosphate buffer to obtain the acellular matrix.

Compared with the prior art, the beneficial effects achieved by the invention are as follows:
The present invention obtains a natural extracellular matrix by cultivating cells and then decellularizing the cells. In this way, various important components and frameworks of the extracellular matrix are retained, which is beneficial to the adhesion of nerve cells and the guidance of axon regeneration, and accelerates the regeneration and functional recovery of peripheral nerves.

The cell matrix nerve graft does not contain exogenous toxic substances brought in by the preparation process and has good biocompatibility, biodegradability, and good mechanical properties.

The use of the human bone marrow mesenchymal stem cells and the serum-free culture medium facilitates the clinical transformation of tissue-engineered nerve grafts.

The acellular matrix is wrapped around the biodegradable scaffold and can self-assemble to form a tubular structure, which provides necessary pathways for the growth of nerve cells and takes the effect of guidance and oriented growth.

The cell matrix nerve graft prepared by the method of the invention avoids the immunogenicity of allogeneic cell transplantation and is suitable for use by large-scale populations.

### BRIEF DESCRIPTION OF THE SEVERAL VIEWS OF THE DRAWINGS

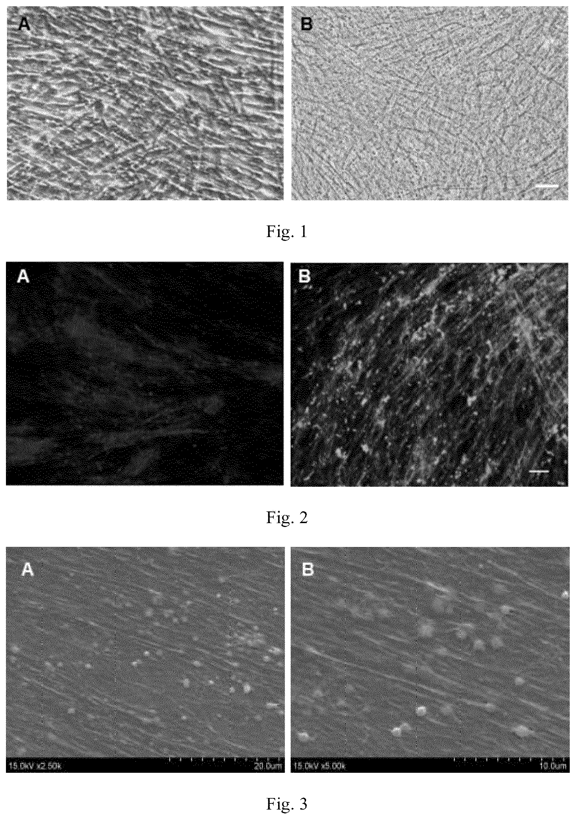

Fig. 1 shows micrographs under a light microscope before and after preparation of an acellular matrix according to an embodiment of the present invention: (A) the micrograph of stem cells under a light microscope before decellularization; (B) the micrograph of the matrix under a light microscope after decellularization;
Fig. 2 shows immunochemistry micrographs of the acellular matrix by Collagen IV staining and Fibronectin staining according to an embodiment of the invention: (A) Collagen IV staining; (B) Fibronectin staining;
Fig. 3 shows SEM micrographs of the acellular matrix according to an embodiment of the invention: (A) micrograph under a 2500x electron microscope; (B) micrograph under a 5000x electron microscope;

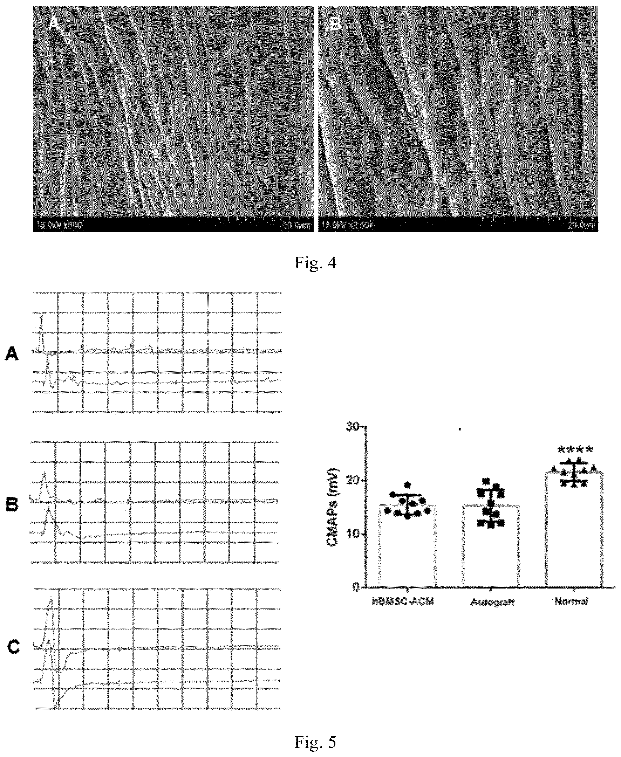

Fig. 4 shows SEM micrographs of a cell matrix nerve graft according to an embodiment of the invention: (A) micrograph under an 800x electron microscope; (B) micrograph under a 2500x electron microscope;
Fig. 5 shows electrophysiological test results of regenerated nerve according to an embodiment of the invention: the diagrams on the left show the composite muscle action potential waveforms of cell matrix group (A), autograft group (B), and sham operation group (C); the diagram on the right shows the comparison of the composite muscle action potential amplitudes of regenerated nerve of different samples in each group, ****p<0.0001;

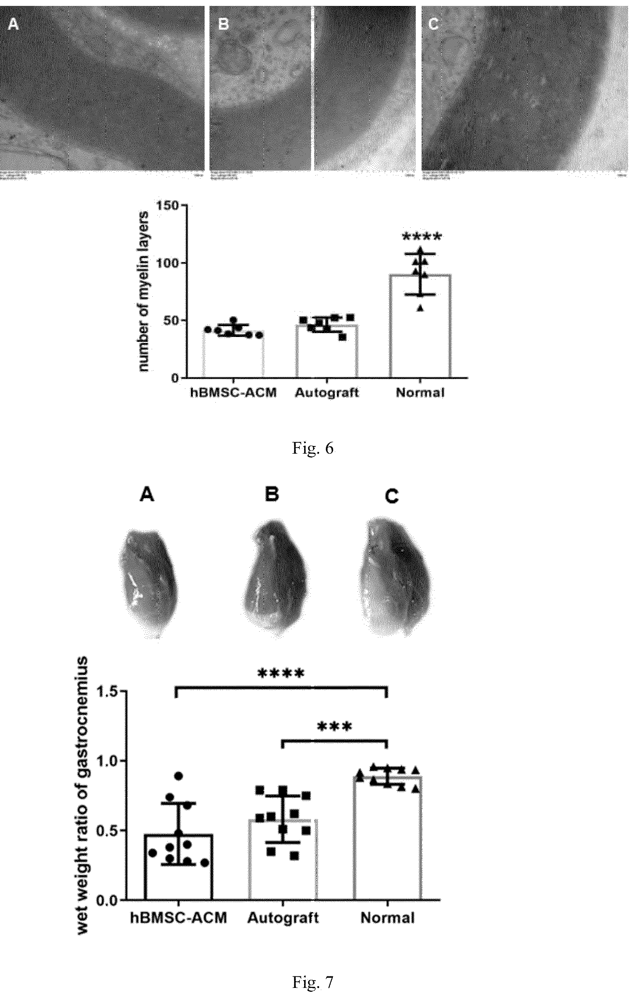

Fig. 6 shows cross-sectional TEM micrographs of regenerated nerve at the middle section according to an embodiment of the invention: partial TEM micrographs of myelin magnified 20,000 times for cell matrix group (A), autograft group (B) and sham operation group (C) are shown above; a comparison chart of the number of myelin layers of the groups is shown below, ****p<0.0001;
Fig. 7 shows wet weight ratio results of target muscle according to an embodiment of the invention: the appearance and morphology of gastrocnemius of cell matrix group (A), autograft group (B) and sham operation group (C) is shown above; wet weight ratio analysis for the groups is shown below, ***p<0.001, ****p<0.0001;

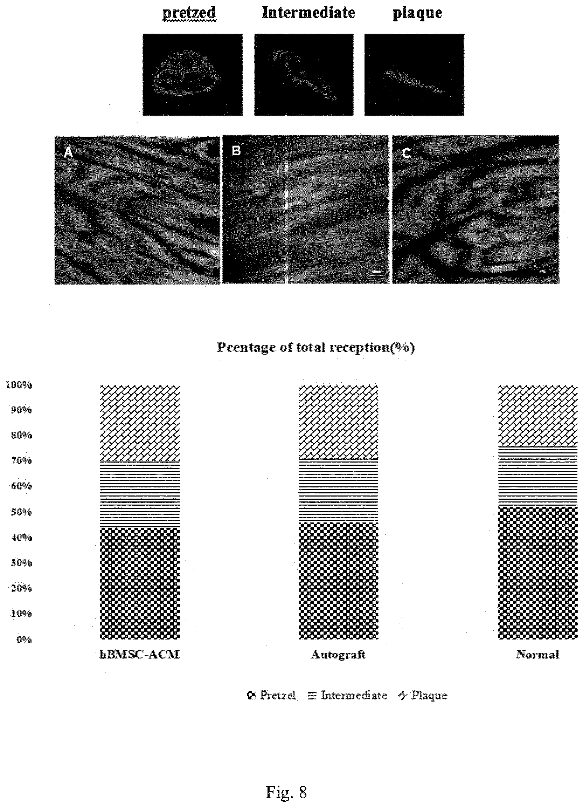

Fig. 8 shows the motor endplate reconstruction results according to an embodiment of the invention: the maturity of motor endplates is shown at the top; an observation diagram of motor endplates of cell matrix group (A), autograft group (B) and sham operation group (C) is shown in the middle; a histogram of statistical analysis of percentage of motor endplates in different stages is shown at the bottom; and

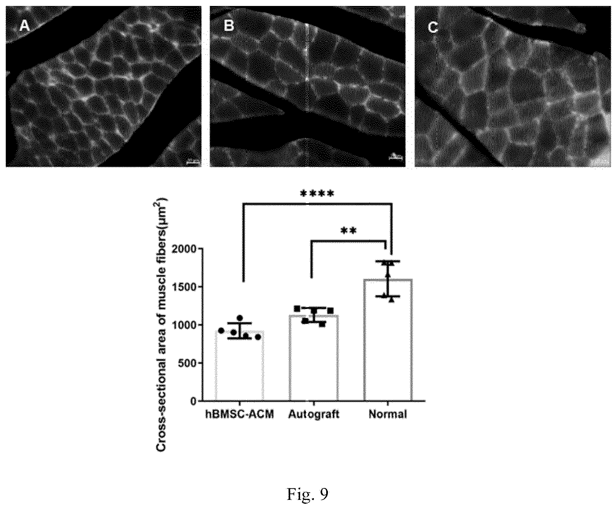

Fig. 9 shows cross-sectional area results of target muscle fibers according to the embodiment of the invention: an observation diagram of the gastrocnemius fiber cross-section of cell matrix group (A), autograft group (B) and sham operation group (C) is shown above; a statistical comparison chart of the groups about the cross-sectional area of gastrocnemius fibers is shown below, **p<0.01, ****p<0.0001.

### DETAILED DESCRIPTION OF THE INVENTION

The invention will be further described with reference to the accompanying drawings. The following embodiments are only for illustrating the technical solution of the invention more clearly, but not for limiting the scope of the invention.

### Embodiment 1

### Preparation of acellular matrix

hBMSCs of 3rd passage (purchased from ATCC cell bank) were seeded into a large dish at 10⁵ cells/ml. When the cell saturation reached 90%, a serum-free medium supplemented with ascorbic acid was added to stimulate the stem cells for 7-14 days to promote the cells to secrete extracellular matrix. In this embodiment, the stem cells were stimulated for 7-14 days.

The cells were then flushed three times with a phosphate buffer, and sterile ultrapure water was added for hypotonic lysis at 37 °C± 0.5 °C for at least 10 min.

The sterile ultrapure water was then removed, and a cell lysis buffer composed of 3% Triton X-100 and 2% SDS was added for extraction at 37 °C ± 0.5 °C no more than 5 min. The cells were then flushed three times with the phosphate buffer to obtain the acellular matrix.

In this embodiment, hBMSCs were stimulated with ascorbic acid for 10 days. As time went by, the extracellular matrix secreted by the cells gradually became thicker. In the later stage, the cells aged, their adhesion properties decreased, and the extracellular matrix was "curled", causing the failure of samples.

The acellular matrix obtained in this embodiment was soaked in the phosphate buffer and stored in a low temperature range of 2-6 °C. The acellular matrix was stored at 4 °C in this embodiment and the storage period can reach about 2 weeks. Fig. 1 (B) is a micrograph of the obtained acellular matrix under a light microscope. Fig. 1 (A) is a micrograph of the stem cell under a light microscope before decellularization.

Some components of the acellular matrix were identified through Collagen IV and Fibronectin immunochemistry: The obtained acellular matrix was fixed with 4% paraformaldehyde at room temperature for 30 min, flushed three times with the phosphate buffer, and then the primary antibodies: rabbit anti Collagen IV (1:100) and sheep anti fibronectin (1:100) were added, and the acellular matrix was incubated overnight at 4 °C.

After flushing three times with the phosphate buffer, secondary antibodies: Cy3-Goat anti-rabbit IgG (H+L) (1:600) and donkey anti-sheep(AF647) (1:500) were added, and the acellular matrix was incubated overnight at 4 °C.

The acellular matrix was flushed three times with the phosphate buffer. After DAPI staining, the acellular matrix was tested under Fluorescence confocal microscope (DMR, Leica). Referring to Fig. 2, it shows Collagen IV and Fibronectin immunochemistry micrographs of the acellular matrix according to the embodiment of the invention: (A) Collagen IV staining; (B) Fibronectin staining.

The invention uses the acellular matrix derived from cultured cells. Compared with tissue-derived acellular matrix, it eliminates the transfer of pathogens during culture and expansion. It can break through the original structural limitations of tissue- or organ-derived extracellular matrix. After combination with tissue engineering biomaterials, controllable biodegradation rates and effective mechanical properties can be achieved while maintaining the required geometry and flexibility of the scaffold.

The cell matrix in the tissue-engineered nerve graft used in the invention was obtained by decellularization after the secretion and formation of cultured cells. A new cell matrix nerve graft was constructed by combining the hBMSC-derived acellular matrix with a biodegradable scaffold. The immunogenicity was reduced or eliminated after transplantation, making it suitable for use by large-scale populations. Moreover, the BMSCs of the invention were human-derived and cultured without serum, laying a solid foundation for the clinical transformation of tissue-engineered nerve grafts.

### Embodiment 2

### SEM testing of acellular matrix

Referring to Fig. 3, it shows SEM micrographs of the acellular matrix according to the embodiment of the invention: (A) micrograph observed under a 2500x electron microscope; (B) micrograph observed under a 5000x electron microscope. SEM results showed that the acellular matrix had a dense fiber network structure, with spheres of different sizes distributed between the network fibers, and the fibers were arranged with certain directionality.

The SEM testing of acellular matrix in this embodiment was performed as follows.

The acellular matrix was placed on a 14 mm round glass slide, fixed with 4% glutaraldehyde in a refrigerator at 4 °C for 2-4 h, and flushed three times with the phosphate buffer, 10 min each time.

The acellular matrix was fixed with 1% osmic acid at room temperature in the dark for 2 h, and then flushed three times with double-distilled water, 10 min each time.

Gradient ethanol dehydration (the ethanol concentrations were 30%, 50%, 70%, 80%, and 95% sequentially) was performed, at least 10 min each time. The acellular matrix was then soaked in absolute ethanol overnight which was replaced with fresh absolute ethanol next day. After absolute ethanol-isoamyl acetate replacement (the ratios were 1:1, 1:2, pure isoamyl acetate), the acellular matrix was dried with a critical point dryer, and plated, and then observed under a Hitachi S-3400II scanning electron microscope.

### Embodiment 3

### Construction of cell matrix nerve graft

The jelly-like membrane-like acellular matrix with a thickness of about 0.12mm, measured with a vernier caliper was rolled around the biodegradable scaffold to three specifications of 6, 9 or 12 layers, and wrapped around the biodegradable scaffold to construct the initial form of the cell matrix nerve graft. The initial form of the cell matrix nerve graft was set still at 2-6 °C for at least 24 h for self-assembly to form a tubular structure. **In** this embodiment, the initial form of the cell matrix nerve graft had self-assembled at 4 °C for 24 h and then was lyophilized at -80 °C to construct the cell matrix nerve graft.

Fig. 4 shows SEM micrographs of the cell matrix nerve graft according to an embodiment of the invention: (A) micrograph observed under an 800x electron microscope; (B) micrograph observed under a 2500x electron microscope. SEM results showed that the graft showed certain directionality, and spheres of different sizes were distributed between the fibers.

The SEM was performed as follows:
The cell matrix nerve graft was cut longitudinally from the middle, fixed with 4% glutaraldehyde in a refrigerator at 4 °C for 2-4 h, and flushed three times with the phosphate buffer, 10 min each time.

The acellular matrix was fixed with 1% osmic acid at room temperature in the dark for 2 h, and then flushed three times with double-distilled water, 10 min each time.

Gradient ethanol dehydration (the ethanol concentrations were 30%, 50%, 70%, 80%, and 95% sequentially) was performed, 10 min each time. The cell matrix nerve graft was then soaked in absolute ethanol overnight which was replaced with fresh absolute ethanol next day. After absolute ethanol-isoamyl acetate replacement (the ratios were 1:1, 1:2, pure isoamyl acetate), the cell matrix nerve graft was dried with a critical point dryer, and plated, and then observed under a Hitachi S-3400II scanning electron microscope.

### Embodiment 4

Repair of sciatic nerve defects in rats using cell matrix nerve graft
The cell matrix nerve graft was used to repair sciatic nerve defects in rats, and nerve regeneration rate and functional recovery of sciatic nerve were tested through electrophysiology, TEM, immunohistochemistry and other methods. The specific procedures were as follows:

First, 10 mm rat sciatic nerve defect models were established and randomly divided into three groups: Group A, Group B, and Group C. Group A used the cell matrix nerve graft to repair sciatic nerve defects in rats and was called the cell matrix group. Group B used autologous nerves to repair sciatic nerve defects in rats and was called the autograft group. Group C exposed the sciatic nerve without making defects and was called the sham operation group. Twelve weeks after surgery, the sciatic nerve on the surgical side was exposed under moderate anesthesia and nerve electrophysiological testing was performed.

Referring to Fig. 5, it shows electrophysiological test results of regenerated nerve according to an embodiment of the invention: the diagrams on the left show the composite muscle action potential waveforms of cell matrix group (A), autograft group (B), and sham operation group (C); the diagram on the right shows the comparison of the composite muscle action potential amplitudes of regenerated nerve in each group, ****p<0.0001. Different shapes of symbols in the figure represent different groups, and the number represents the number of samples. It can be seen from the figure that the average CMAP amplitudes of the cell matrix group, the autograft group and the sham operation group are: 15.49 ± 1.82mV, 15.33 ± 2.98mV, and 21.56 ± 1.67mV, respectively. There is no statistical difference between the cell matrix group and the autograft group (P>0.05).

The middle section of the regenerated nerve was taken for TEM examination. Referring to Fig. 6, it shows cross-sectional TEM micrographs of regenerated nerve at the middle section according to an embodiment of the invention: partial TEM micrographs of myelin magnified 20,000 times for cell matrix group (A), autograft group (B) and sham operation group (C) are shown above; a comparison chart of the number of myelin layers of the groups is shown below, ****p<0.0001. It can be seen from the figure that the number of myelin layers in the cell matrix group, the autograft group and the sham operation group are: 41 ± 5, 46 ± 6, and 90 ± 17, respectively. There is no statistical difference between the cell matrix group and the autograft group (P>0.05).

The wet weight ratio of the target muscle and the testing of motor endplates after surgery are important indicators for evaluating the reconstruction of the innervation function of the target muscle by regenerated nerve. Early muscle denervation occurs, and as time goes by, regenerated nerve will re-innervate the target muscle. Referring to Fig. 7, it shows wet weight ratio results of target muscle according to an embodiment of the invention: the appearance and morphology of gastrocnemius of cell matrix group (A), autograft group (B) and sham operation group (C) is shown above; wet weight ratio analysis for the groups is shown below, ***p<0.001, ****p<0.0001. It can be seen from the figure that 12 weeks after the surgery, the gastrocnemius wet weight ratios of the cell matrix group, the autograft group and the sham operation group are: 0.48 ± 0.22, 0.58 ± 0.16, 0.88 ± 0.06, respectively. There is no statistical difference between the cell matrix group and the autograft group (P>0.05).

The motor endplate is the neuromuscular junction between the nerve terminal and the target muscle it innervates. It has a synaptic structure. α-bungarotoxin specifically labels acetylcholine receptors on the postsynaptic membrane. The gastrocnemius muscle on the surgical side was taken from the rats in the cell matrix group, the autograft group and the sham operation group 12 weeks after bridging. They were fixed and dehydrated and then frozen and sectioned longitudinally. The motor endplates were stained and their maturity was analyzed.

The maturity of the motor endplates includes different stages: (1) Early stage: "plaque", with small size and non-porous structure; (2) Mature stage: "pretzel", with large size and reticular and porous structure; (3) Middle stage: "intermediate" (between mature stage and early stage). Fig. 8 shows the motor endplate reconstruction results according to an embodiment of the invention: the maturity of motor endplates is shown at the top, where Pretzel means mature stage, Intermediate means transitional stage, and Plaque means immature stage; an observation diagram of motor endplates of cell matrix group (A), autograft group (B) and sham operation group (C) is shown in the middle; a histogram of statistical analysis of percentage of motor endplates in different stages is shown at the bottom.

The motor endplates in the sham operation group were mostly in the mature stage with large butterfly-wing-shaped plaques and reticular and porous structure. The motor endplates innervated by regenerated nerves in the cell matrix group and the autolograft group were small and slender. Quantitative statistics of motor endplates 12 weeks after surgery showed that compared with the other two groups, most of the motor endplates in the sham operation group were in the mature stage (52%), and the percentage of motor endplates in the mature stage of the cell matrix group (44%) was not statistically significantly different from that of the autograft group (46%) (P>0.05).

Referring to Fig. 9, it shows cross-sectional area results of target muscle fibers according to an embodiment of the invention. The gastrocnemius muscle on the surgical side was taken from the rats in the cell matrix group, the autograft group and the sham operation group 12 weeks after bridging. They were fixed and dehydrated and then frozen and transected. The extracellular matrix of the muscle fibers was stained with Laminin. In the figure, an observation diagram of the gastrocnemius fiber cross-section of cell matrix group (A), autograft group (B) and sham operation group (C) is shown above; a statistical comparison chart of the groups about the cross-sectional area of gastrocnemius fibers is shown below, **p<0.01, ****p<0.0001. As can be seen from the figure, the cross-sectional areas of muscle fibers from the cell matrix group, the autograft group and the sham operation group are: 923.4 ± 98.85 µm², 1131 ± 92.23 µm², and 1606 ± 229.9 µm². There is no statistical difference between the cell matrix group and the autograft group (P>0.05).

The cell matrix nerve graft used in the present does not contain exogenous toxic substances brought in by the preparation process and has good biocompatibility, biodegradability, and good mechanical properties. Use of the biodegradable scaffold and the formation of tubular structure provide the necessary pathways and necessary guidance and take the effect of guidance and oriented growth. The acellular matrix used can effectively promote nerve regeneration and functional recovery.

In view of the defects in the prior art, the invention provides a cell matrix nerve graft that is beneficial to cell adhesion and migration and can promote nerve regeneration and a preparation method thereof. In vitro decellularization technology is used to remove stem cells to obtain an acellular matrix, which is then wrapped around a biodegradable scaffold to form a cell matrix nerve graft to repair peripheral nerve defects. The invention can overcome the shortcomings of autologous nerve transplantation, avoid the immunogenicity of allogeneic cell transplantation, provide an acellular matrix with a certain orientation that is conducive to the proliferation and migration of nerve cells, and establish a local microenvironment that is conducive to the growth of regenerated axons to achieve the ideal goals of rapid nerve growth and functional recovery, thereby providing a feasible solution for clinical treatment.

## Claims

1. A cell matrix nerve graft for repairing peripheral nerve injury, comprising acellular matrix and a scaffold,
**characterized in that**:
wherein the acellular matrix is obtained by decellularization after secretion and formation of stem cells, and then the acellular matrix is rolled up to multiple layers with the scaffold as an axis and wrapped around the scaffold, then is set at 2-6 °C for at least 24 h for self-assembly to form a tubular structure; wherein the scaffold is biodegradable.

2. The cell matrix nerve graft for repairing peripheral nerve injury according to claim 1, wherein the stem cells are human bone marrow mesenchymal stem cells (hBMSCs).

3. The cell matrix nerve graft for repairing peripheral nerve injury according to claim 1, wherein the scaffold comprises chitosan and recombinant human collagen.

4. A preparation method of the cell matrix nerve graft for repairing peripheral nerve injury according to any one of claims 1-3, comprising the following steps:
preparing the acellular matrix;
rolling the acellular matrix up to multiple layers with the scaffold as an axis and wrapping the acellular matrix around a scaffold to construct an initial form of the cell matrix nerve graft;
setting the initial form of the cell matrix nerve graft still at 2-6 °C for at least 24 h for self-assembly to form a tubular structure; and
lyophilizing the tubular structure to construct the cell matrix nerve graft.

5. The preparation method of the cell matrix nerve graft for repairing peripheral nerve injury according to claim 4, wherein the tubular structure is lyophilized at -80 °C.

6. The preparation method of the cell matrix nerve graft for repairing peripheral nerve injury according to claim 4, wherein the acellular matrix is rolled up to 6, 9 or 12 layers with the biodegradable scaffold as an axis and wrapped around the biodegradable scaffold.

7. The preparation method of the cell matrix nerve graft for repairing peripheral nerve injury according to claim 4, wherein the acellular matrix is prepared as follows:
diluting hBMSCs, seeding the hBMSCs into a large dish, and when the cell saturation reaches 90% or above, adding a serum-free culture medium supplemented with ascorbic acid to stimulate the stem cells for 7-14 days; and
flushing with a phosphate buffer, adding sterile ultrapure water for hypotonic lysis, removing the sterile ultrapure water, adding a cell lysis buffer for extraction, and then flushing with the phosphate buffer to obtain the acellular matrix.

## Patentansprüche

1. Ein Zellmatrix- -Nerventransplantat zur Reparatur von peripheren Nervenverletzungen , umfassend eine azelluläre Matrix und ein Gerüst,
**dadurch gekennzeichnet, dass**:
wobei die azelluläre Matrix durch Dekellularisierung nach Sekretion und Bildung von Stammzellen gewonnen wird, und anschließend die azelluläre Matrix mit dem Gerüst als Achse zu mehreren Schichten aufgerollt und um das Gerüst gewickelt wird, woraufhin sie mindestens 24 Stunden lang bei 2-6 °C zur Selbstorganisation belassen wird, um eine röhrenförmige Struktur zu bilden; wobei das Gerüst biologisch abbaubar ist.

2. Das Zellmatrix-Nerventransplantat zur Reparatur peripherer Nervenverletzungen gemäß Anspruch 1, wobei die Stammzellen menschliche mesenchymale Stammzellen aus dem Knochenmark (hBMSCs) sind.

3. Zellmatrix-Nerventransplantat zur Reparatur peripherer Nervenverletzungen gemäß Anspruch 1, wobei das Gerüst Chitosan und rekombinantes humanes Kollagen umfasst.

4. Verfahren zur Herstellung des Zellmatrix-Nerventransplantats zur Reparatur peripherer Nervenverletzungen gemäß einem der Ansprüche 1 bis 3, umfassend die folgenden Schritte:
Herstellen der azellulären Matrix;
Aufrollen der azellulären Matrix zu mehreren Schichten mit dem Gerüst als Achse und Umwickeln der azellulären Matrix um ein Gerüst, um eine Ausgangsform des Zellmatrix-Nerventransplantats zu bilden;
Halten der Ausgangsform des Zellmatrix-Nerventransplantats bei 2-6 °C für mindestens 24 Stunden zur Selbstorganisation, um eine röhrenförmige Struktur zu bilden; und
Gefriertrocknen der röhrenförmigen Struktur, um das Zellmatrix-Nerventransplantat herzustellen.

5. Verfahren zur Herstellung des Zellmatrix-Nerventransplantats zur Reparatur einer peripheren Nervenverletzung gemäß Anspruch 4, wobei die röhrenförmige Struktur bei -80 °C lyophilisiert wird.

6. Verfahren zur Herstellung des Zellmatrix-Nerventransplantats zur Reparatur peripherer Nervenverletzungen gemäß Anspruch 4, wobei die azelluläre Matrix mit dem biologisch abbaubaren Gerüst als Achse zu 6, 9 oder 12 Schichten aufgerollt und um das biologisch abbaubare Gerüst gewickelt wird.

7. Verfahren zur Herstellung eines Zellmatrix-Nerventransplantats zur Reparatur peripherer Nervenverletzungen gemäß Anspruch 4, wobei die azelluläre Matrix wie folgt hergestellt wird:
Verdünnen von hBMSCs, Ausplattieren der hBMSCs in eine große Schale und, wenn die Zellbesatzdichte 90 % oder mehr erreicht, Zugabe eines mit Ascorbinsäure versetzten serumfreien Kulturmediums, um die Stammzellen 7-14 Tage lang zu stimulieren; und
Spülen mit einem Phosphatpuffer, Zugabe von sterilem ultrareinem Wasser zur hypotonischen Lyse, Entfernen des sterilen ultrareinen Wassers, Zugabe eines Zelllysepuffers zur Extraktion und anschließendes Spülen mit dem Phosphatpuffer, um die azelluläre Matrix zu erhalten.

## Revendications

1. Greffe nerveuse à matrice cellulaire destinée à la réparation d'une lésion nerveuse périphérique , comprenant une matrice acellulaire et un support,
**caractérisée en ce que** :
la matrice acellulaire est obtenue par décellularisation après sécrétion et formation de cellules souches , puis la matrice acellulaire est enroulée en plusieurs couches autour de l'échafaudage servant d'axe et enveloppée autour de celui-ci, puis est maintenue à une température comprise entre 2 et 6 °C pendant au moins 24 h pour permettre l'auto-assemblage et former une structure tubulaire ; l'échafaudage étant biodégradable.

2. Greffe nerveuse à matrice cellulaire pour la réparation d'une lésion nerveuse périphérique selon la revendication 1, dans laquelle les cellules souches sont des cellules souches mésenchymateuses de moelle osseuse humaine (hBMSC).

3. Greffe nerveuse à matrice cellulaire destinée à réparer une lésion nerveuse périphérique selon la revendication 1, dans laquelle le support comprend du chitosane et du collagène humain recombinant.

4. Procédé de préparation de la greffe nerveuse à matrice cellulaire destinée à réparer une lésion nerveuse périphérique selon l'une quelconque des revendications 1 à 3, comprenant les étapes suivantes :
la préparation de la matrice acellulaire ;
enrouler la matrice acellulaire en plusieurs couches autour de la structure de support et envelopper la matrice acellulaire autour de la structure de support pour construire une forme initiale de la greffe nerveuse à matrice cellulaire ;
maintien de la forme initiale de la greffe nerveuse à matrice cellulaire à une température comprise entre 2 et 6 °C pendant au moins 24 h pour permettre l'auto-assemblage en vue de former une structure tubulaire ; et
lyophiliser la structure tubulaire pour constituer la greffe nerveuse à matrice cellulaire.

5. Procédé de préparation de la greffe nerveuse à matrice cellulaire destinée à réparer une lésion nerveuse périphérique selon la revendication 4, dans lequel la structure tubulaire est lyophilisée à -80 °C.

6. Procédé de préparation de la greffe nerveuse à matrice cellulaire pour la réparation d'une lésion nerveuse périphérique selon la revendication 4, dans lequel la matrice acellulaire est enroulée en 6, 9 ou 12 couches avec le support biodégradable comme axe et enroulée autour du support biodégradable.

7. Procédé de préparation de la greffe nerveuse à matrice cellulaire pour la réparation de lésions nerveuses périphériques selon la revendication 4, dans lequel la matrice acellulaire est préparée comme suit :
diluer les hBMSC, ensemencer les hBMSC dans une grande boîte de culture, et lorsque la saturation cellulaire atteint 90 % ou plus, ajouter un milieu de culture sans sérum enrichi en acide ascorbique pour stimuler les cellules souches pendant 7 à 14 jours ; et
rincer avec un tampon phosphate, ajouter de l'eau ultra-pure stérile pour la lyse hypotonique, retirer l'eau ultra-pure stérile, ajouter un tampon de lyse cellulaire pour l'extraction, puis rincer avec le tampon phosphate pour obtenir la matrice acellulaire.
